# EUROPEAN PATENT APPLICATION

(11) **EP 4 603 118 A1**
(43) Date of publication of application: **20.08.2025**
(21) Application number: 23883076.4
(22) Date of filing: 25.10.2023
(51) Int. Cl.: A61M 5/145, A61M 5/172, A61M 5/142, G16H 20/17, G16H 50/20, G16H 40/63

(54) **DRUG INJECTION DEVICE AND METHOD**

(30) Priority: 25.10.2022 KR 20220138208; 24.10.2023 KR 20230142851
(71) Applicant: Caremedi Co., Ltd., Seoul 07207 (KR); Sogang University Research & Business Development Foundation, Seoul 04107 (KR)
(72) Inventor: SHIN, Woonsup, Seoul 04065 (KR); ZHU, Enhua, Seoul 04109 (KR); SHIN, Yeong Jong, Gimpo-si Gyeonggi-do 10126 (KR)
(74) Representative: Manitz Finsterwald Patent- und Rechtsanwaltspartnerschaft mbB
(86) International application number: PCT/KR2023/016597
(87) International publication number: WO 2024/090971

(57) **Abstract**

A drug injection device according to the present disclosure includes: a drug injector configured to electrochemically drive an electroosmotic pump to suck a drug from a drug storage and discharge the sucked drug to an injection target; and a controller configured to output, to the drug injector, a control signal corresponding to an injection sequence determined according to an injection mode, wherein when a predetermined event occurs, the controller stops an operation based on a first speed-based injection sequence for a basal injection mode and outputs a control signal corresponding to a second speed-based injection sequence for a temporary basal injection mode, and the electroosmotic pump alternately generates negative pressure and positive pressure in response to the control signal to suck and discharge the drug.

## Description

### TECHNICAL FIELD

The technical field of the present disclosure relates to a drug injection device and a drug injection method.

### BACKGROUND

Drugs may be injected into the body in various ways, such as oral, subcutaneous, and intravenous administration, depending on the type of drug and the purpose and method of treatment. A drug injector using a drug pump may automatically inject a drug into the body at a desired speed and dose at a required time. Therefore, a drug injector using a drug pump may be used not only in hospitals or in patients' daily living environments, but also in various forms.

An insulin pump, which is commonly called an insulin injector, is a medical device that supplies insulin into the body from the outside at a predetermined time to accurately control the blood sugar level, similar to the pancreas, for a diabetic patient who does not secrete insulin or secretes only a small amount of insulin.

Such an insulin pump is used by insulin-dependent diabetic patients, and may continuously inject a drug for 24 hours in a state of being attached to the patient. In this way, the insulin injector needs to periodically inject a drug into a diabetic patient for a long period of time. Therefore, technological development is actively underway to miniaturize and automate insulin injectors for the convenience of users.

In general, the insulin injector operates in a basal injection mode in which a drug is injected into a user for a predetermined period of time to maintain the user's blood sugar level consistently. The basal injection mode is set based on the user's usual activity level. However, when the user's activity level changes due to exercise or rest, insulin injection cannot be flexibly adjusted.

This may cause an increase or decrease in the user's blood sugar level. Therefore, a method for continuously injecting a drug into a patient, stably injecting an accurate amount of the drug depending on the patient's activity level, and controlling drug injection to suppress blockage in a flow path during the drug injection is required.

### DISCLOSURE OF THE INVENTION

### PROBLEMS TO BE SOLVED BY THE INVENTION

In view of the foregoing, the present disclosure is conceived to provide a method of drug injection in a temporary basal injection mode by a drug injection device based on an electroosmotic pump.

The problems to be solved by the present disclosure are not limited to the above-described problems. There may be other problems to be solved by the present disclosure.

### MEANS FOR SOLVING THE PROBLEMS

As a means for solving the above-described problems, a drug injection device according to an embodiment of the present disclosure includes: a drug injector configured to electrochemically drive an electroosmotic pump to suck a drug from a drug storage and discharge the sucked drug to an injection target; and a controller configured to output, to the drug injector, a control signal corresponding to a speed-based injection sequence determined according to an injection mode, wherein when a predetermined event occurs, the controller stops an operation based on a first speed-based injection sequence for a basal injection mode and outputs a control signal corresponding to a second speed-based injection sequence for a temporary basal injection mode, and the electroosmotic pump alternately generates negative pressure and positive pressure in response to the control signal to suck and discharge the drug.

A drug injection method according to an embodiment of the present disclosure includes: a process of setting a speed-based injection sequence determined according to an injection mode when a predetermined event occurs; a process of applying a control signal corresponding to the speed-based injection sequence to the electroosmotic pump; and a process of causing the electroosmotic pump to alternately generate negative pressure and positive pressure for each pulse block in response to the control signal to suck and discharge a drug, wherein the process of applying the control signal to the electroosmotic pump includes stopping an operation based on a first speed-based injection sequence for a basal injection mode and outputting a control signal corresponding to a second speed-based injection sequence for a temporary basal injection mode.

### EFFECTS OF THE INVENTION

According to the means for solving the problems of the present disclosure, it is possible to control a drug injector including an electroosmotic pump to inject a predetermined amount of drug by setting a speed-based injection sequence based on temporary basal injection conditions.

Also, it is possible to control the drug injector to increase or decrease the amount of drug to be injected for a basal injection mode over a predetermined period of time based on changes in a user's blood sugar level or activity level in a temporary basal injection mode during a basal injection mode operation. Thus, it is possible to maintain the blood sugar level of the user.

### BRIEF DESCRIPTION OF THE DRAWINGS

**FIG.** 1 is a block diagram schematically illustrating a drug injection device according to an embodiment of the present disclosure.
**FIG. 2** is a block diagram schematically illustrating a configuration of a drug injector illustrated in **FIG. 1****.**
**FIG. 3** is a conceptual diagram schematically showing a speed-based injection sequence according to an embodiment of the present disclosure.
**FIG. 4** is an example diagram illustrating a signal structure for the speed-based injection sequence according to an embodiment of the present disclosure.
**FIG. 5** is a table showing an example of a plurality of speed-based injection sequences according to an embodiment of the present disclosure.
**FIG. 6** is a table showing an example of a plurality of pulse blocks according to an embodiment of the present disclosure.
**FIG. 7** is a table showing an example of a speed-based injection sequence structure according to an embodiment of the present disclosure.
**FIG. 8** is an example diagram illustrating a signal structure for a first speed-based injection sequence for a basal injection mode according to an embodiment of the present disclosure.
**FIG. 9** is an example diagram illustrating a signal structure in which a second speed-based injection sequence for a temporary basal injection mode is applied to the first speed-based injection sequence illustrated in **FIG. 8****.**
**FIG. 10** is a block diagram schematically illustrating a configuration of an electroosmotic pump illustrated in **FIG. 2****.**
**FIG. 11** is a conceptual diagram schematically illustrating a configuration of a drug injector illustrated in **FIG. 10****.**
**FIG. 12** is an example diagram schematically illustrating an operation of a driver illustrated in **FIG. 11****.**
**FIG. 13** illustrates an example of application of the drug injection device according to an embodiment of the present disclosure.
**FIG. 14** is a block diagram schematically illustrating a configuration of the drug injection device illustrated in **FIG. 13****.**
**FIG. 15** **is** a flowchart illustrating a drug injection method according to an embodiment of the present disclosure.
**FIG. 16** is a flowchart for describing a process of setting a speed-based injection sequence illustrated in **FIG. 15****.**
**FIG. 17** is a flowchart for describing the process of setting the speed-based injection sequence illustrated in **FIG. 15****.**
**FIG. 18** is a flowchart for describing the process of setting the speed-based injection sequence illustrated in **FIG. 15****.**

### BEST MODE FOR CARRYING OUT THE INVENTION

Hereafter, embodiments will be described in detail with reference to the accompanying drawings. However, it is to be noted that the present disclosure is not limited to the embodiments but can be embodied in various other ways. Also, the accompanying drawings are provided only for a better understanding of the embodiments disclosed in the present disclosure and are not intended to limit technical ideas disclosed in the present disclosure. In the drawings, parts irrelevant to the description are omitted for the simplicity of explanation, and the size, form and shape of each component illustrated in the drawings can be modified in various ways. Like reference numerals denote like parts throughout the whole document.

Suffixes "module" and "unit" used for components disclosed in the following description are merely intended for easy description of the specification, and the suffixes themselves do not give any special meaning or function. Further, in the following description of the present disclosure, a detailed explanation of known related technologies may be omitted to avoid unnecessarily obscuring the subject matter of the present disclosure.

Throughout the whole document, the term "connected to (contacted with or coupled to)" may be used to designate a connection or coupling of one element to another element and includes both an element being "directly connected to (contacted with or coupled to)" another element and an element being "indirectly connected to (contacted with or coupled to)" another element via another element. Further, through the whole document, the term "comprises or includes" and/or "comprising or including" used in the document means that one or more other components, steps, operation and/or existence or addition of elements are not excluded in addition to the described components, steps, operation and/or elements unless context dictates otherwise.

Further, in describing components of the present disclosure, ordinal numbers such as first, second, etc. can be used only to differentiate the components from each other, but do not limit the sequence or relationship of the components. For example, a first component of the present disclosure may also be referred to as a second component and vice versa.

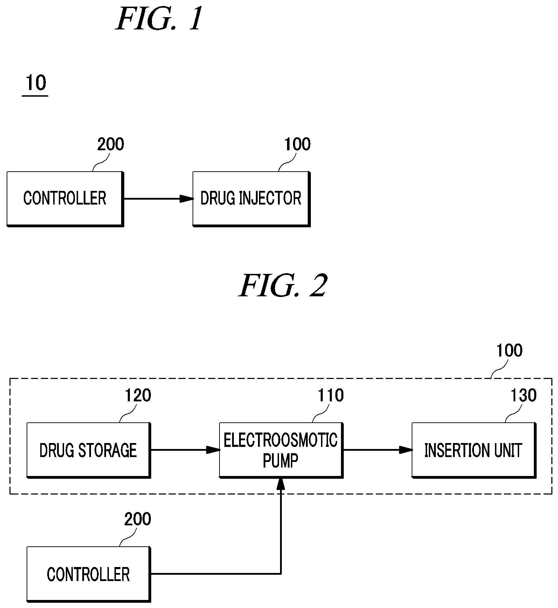

**FIG.** 1 is a block diagram schematically illustrating a drug injection device according to an embodiment of the present disclosure, and **FIG.** 2 is a block diagram schematically illustrating a configuration of a drug injector illustrated in **FIG. 1****.**

Referring to **FIG. 1** and **FIG. 2****,** a drug injection device 10 according to an embodiment of the present disclosure includes a drug injector 100 and a controller 200.

The drug injector 100 electrochemically drives an electroosmotic pump 110 to suck a drug from a drug storage and discharges the sucked drug to an injection target.

The controller 200 outputs, to the drug injector 100, a control signal corresponding to a speed-based injection sequence determined according to an injection mode. If a predetermined event occurs while the drug injector 100 operates in a basal injection mode, which causes the injection mode to be switched to a temporary basal injection mode, the controller 200 temporarily stops an operation based on a first speed-based injection sequence for the basal injection mode and outputs a control signal corresponding to a second speed-based injection sequence for the temporary basal injection mode. The first and second speed-based injection sequences are injection sequences set based on a drug injection speed.

Herein, the basal injection mode is a drug injection mode to inject a drug into the user over a predetermined period of time to maintain the blood sugar level, and the temporary basal injection mode is a drug injection mode to control the amount of drug to be injected for the basal injection mode to be decreased or increased over a predetermined period of time based on changes in the user's blood sugar level or activity level.

The controller 200 may refer to a data processor which is embedded in hardware and includes a physically structured circuit to perform a function expressed as a code or command included in a program. The data processor embedded in hardware may include, for example, a microprocessor, a central processing unit (CPU), a processor core, a multiprocessor, an application-specific integrated circuit (ASIC), a field programmable gate array (FPGA), a micro control unit (MCU), an embedded processor, and the like, but the scope of the present disclosure is not limited thereto.

Meanwhile, the controller 200 may be embedded solely in the drug injection device 10, or may control the temporary basal injection mode of the drug injection device 10 by being connected to a user device 40, which will be described later, through a communication module and being integrated into the user device 40.

Hereafter, the speed-based injection sequence will be described. The speed-based injection sequence is configured by arranging a plurality of pulse blocks 20 that satisfies a drug injection speed based on drug injection conditions including a drug injection period and the drug injection speed. Herein, the speed-based injection sequence is configured by arranging a plurality of pulse blocks that satisfies a drug injection speed for a unit time (e.g., one hour or 30 minutes) and repeating the plurality of pulse blocks during the drug injection period. Herein, the speed-based injection sequence is equally applied to both the first speed-based injection sequence for the basal injection mode and the second speed-based injection sequence for the temporary basal injection mode.

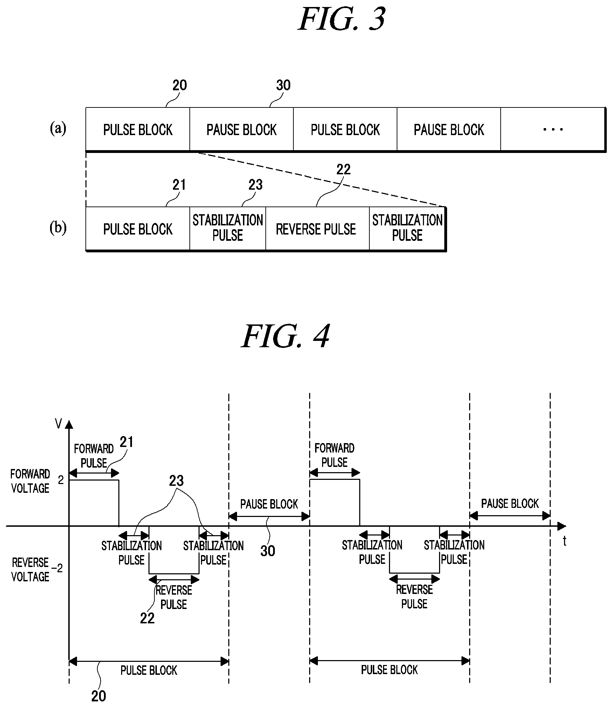

**FIG. 3** is a conceptual diagram schematically showing a speed-based injection sequence according to an embodiment of the present disclosure, and **FIG.** 4 is an example diagram illustrating a signal structure for the speed-based injection sequence according to an embodiment of the present disclosure.

Referring to **FIG. 3** and **FIG. 4****,** the speed-based injection sequence is composed of the plurality of pulse blocks 20 and a plurality of pause blocks 30, and it starts with a pulse block 20, followed by a pause block 30. Each pulse block 20 defines a voltage pulse or a current pulse to be applied to the electroosmotic pump 110, and the amount of drug to be injected varies depending on its duration. Each pause block 30 maintains a voltage of 0 V or a current of 0 A for a predetermined period of time after the pulse block 20 is applied.

To satisfy the drug injection conditions including the drug injection period and the drug injection speed, the speed-based injection sequence is set as follows. First, pulse blocks that satisfy a drug injection amount per unit time need to be arranged to satisfy the drug injection speed. In the present disclosure, various pulse block can be set by using the electroosmotic pump 110. However, during the drug injection period, a plurality of or a predetermined number of pulse blocks need to be arranged whenever possible to minimize a pause period of a pause block arranged after each pulse block and suppress drug drying or blockage. To satisfy these conditions, the speed-based injection sequence may be set as follows.

The speed-based injection sequence is configured by arranging M number of pulse blocks 20 (M is a natural number less than or equal to N) selected from among N number of pulse blocks 20 (N is a natural number), which supply different amounts of drug, based on a drug injection speed per unit time. The plurality of pulse blocks 20 and pause blocks 30 of the speed-based injection sequence are arranged such that a duration of each pause block 30 is less than a recommended pause period while satisfying the drug injection speed. Herein, the number of pulse blocks 20 arranged in the speed-based injection sequence may be a predetermined number per unit time or a maximum number that represents the drug injection speed.

Further, the sum of the durations of the plurality of pulse blocks 20 and the plurality of pause blocks 30 following the pulse blocks 20 of the speed-based injection sequence is set to correspond to the unit time. The pulse blocks 20 are arranged such that a pulse block 20 supplying the largest amount of drug is output first, and the arrangement of subsequent pulse blocks 20 may vary depending on the choice of implementation.

Also, the M number of pulse blocks 20 of the speed-based injection sequence may be set to satisfy the drug injection speed while ensuring that the total number of times the selected pulse blocks 20 are used matches a predetermined number or a maximum number and a duration of the pause block 30 following each pulse block 20 is less than the recommended pause period.

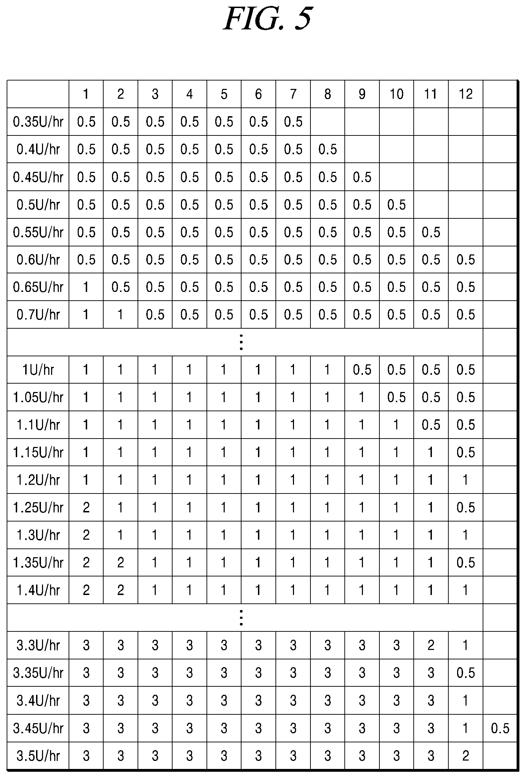

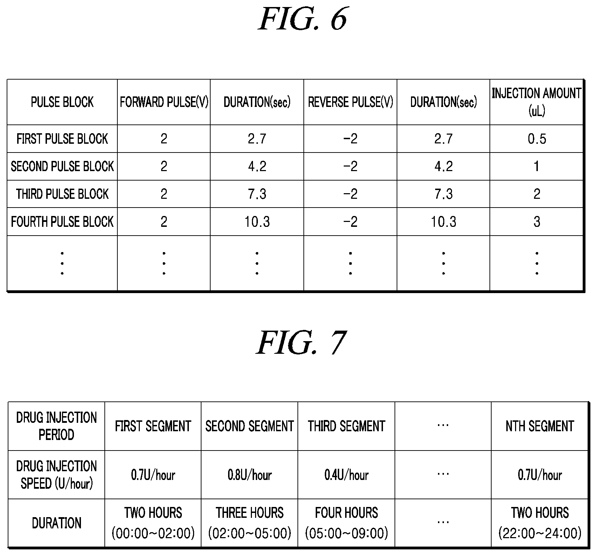

**FIG. 5** is a table showing an example of a plurality of speed-based injection sequences, and **FIG. 6** is a table showing an example of N number of pulse blocks which inject different amounts of drug. The speed-based injection sequence will be described with reference to **FIG. 5** and **FIG. 6****.**

A plurality of speed-based injection sequences shown in **FIG. 5** is set to have different drug injection speeds. Each speed-based injection sequence is composed of the M number of pulse blocks 20 selected from among the N number of pulse blocks 20, which supply different amounts of drug, shown in **FIG. 6**, and satisfying the drug injection speed while ensuring that a duration of each pause block 30 is less than the recommended pause period. Further, in each speed-based injection sequence, the pulse blocks 20 are arranged such that the pulse block 20 supplying the largest amount of drug is output first.

Herein, the speed-based injection sequence is set to include 12 pulse blocks 20. As all the 12 pulse blocks 20 are arranged, it is composed of a combination of the pulse blocks 20 that satisfy the drug injection speed. For example, a speed-based injection sequence with a drug injection speed of 0.7 U/hr is composed of a combination of two pulse blocks 20 supplying 0.5 µL and 1 µL, respectively. The pulse block 20 supplying 1 µL is arranged first. The total number of times the pulse blocks 20 supplying 0.5 µL and 1 µL are used is preset to 12. In **FIG. 5**, the drug injection amount of the speed-based injection sequence is expressed in terms of U, while in **FIG. 6**, the drug injection amount of the pulse blocks 20 is expressed in terms of µL. Since 1 U is equivalent to 10 µL, 0.35 U is equivalent to 3.5 µL.

Although not explicitly shown in the table, a pause block 30 is arranged after each pulse block 20, and to suppress blockage, a duration of the pause block 30 is set to be less than the recommended pause period. The duration of the pause block 30 varies depending on a duration of the preceding pulse block 20.

Referring to **FIG. 5****,** in an example of setting a duration of the pause block 30, each speed-based injection sequence is configured with 12 pulse blocks 20, and, thus, a duration of one pulse block 20 and one pause block 30 is set to 300 seconds (3600 seconds/12). For example, if a pause block 30 follows a pulse block 20 that injects 0.5 µL, a duration of the pause block 30 is determined by subtracting a duration of the pulse block 20 from 300 seconds. Referring to **FIG. 6****,** the duration of the pulse block 20 that injects 0.5 µL is 5.4 seconds (*i.e.,* the sum of durations of a forward pulse and a reverse pulse), and, thus, the duration of the pause block 30 is set to 294.6 seconds (*i.e.,* 300 seconds - 5.4 seconds).

Further, there may be cases where a speed-based injection sequence does not include a combination of a predetermined number of pulse blocks 20. In such cases, if the number of pulse blocks 20 is smaller or greater than the predetermined number, speed-based injection sequences with drug injection speeds between 0.35 U/hr and 0.55 U/hr are composed of fewer than 12 pulse blocks 20 as shown in **FIG. 5****.** In such cases, the speed-based injection sequences are composed of the maximum possible number smaller than the predetermined number of pulse blocks 20 to satisfy the drug injection speed. For example, a speed-based injection sequence with a speed of 3.45 U/hr may be composed of 13 pulse blocks 20, *i.e.,* the number of pulse blocks 20 exceeds 12. In this case, the speed-based injection sequence may be reset such that pulse blocks 20 and pause blocks 30 are arranged by arranging 13 pulse blocks 20 per hour.

Although **FIG. 5** shows an example where the unit time of the speed-based injection sequence is set to one hour, the present disclosure is not limited thereto. The number and configuration of pulse blocks 20 in the speed-based injection sequence may vary depending on a predetermined unit time.

The speed-based injection sequences are continuously arranged throughout the drug injection period. Specifically, the drug injection period may be divided into at least one segment, and each segment includes information about a duration and a drug injection speed for the duration. Each segment is configured by repeatedly arranging the speed-based injection sequence set for the unit time to satisfy the drug injection speed during its duration. Herein, the duration and drug injection speed of each segment may be set by the user.

**FIG. 7** is a table showing an example of a speed-based injection sequence structure according to an embodiment of the present disclosure. The speed-based injection sequence composed of a plurality of segments will be described with reference to **FIG. 7****.** In **FIG. 7****,** the drug injection period is set to 24 hours, and this 24-hour period is divided into a plurality of segments, each assigned a specific time slot. For example, a first segment refers to a segment in which the drug is injected at a speed of 0.7 U/hour for two hours. This means that two speed-based injection sequences with a drug injection speed of 0.7 U/hour as shown in **FIG. 5** are continuously arranged. Herein, the unit time of each segment is set to one hour, but the present disclosure is not limited thereto and segments may be divided by a predetermined unit time. Also, the first speed-based injection sequence for the basal injection mode may be configured as shown in **FIG. 7****.**

Hereafter, the pulse block 20 will be described. Each pulse block 20 includes information about a pair of pulse signals including a forward pulse and a reverse pulse, and includes information about an amplitude of each pulse and a duration for which a pulse is maintained. The pair of pulse signals including the forward pulse and the reverse pulse are applied to the electroosmotic pump 110 to alternately generate negative pressure and positive pressure. Thus, negative pressure and positive pressure are alternately generated for each of the pulse blocks 20 to suck and discharge a drug.

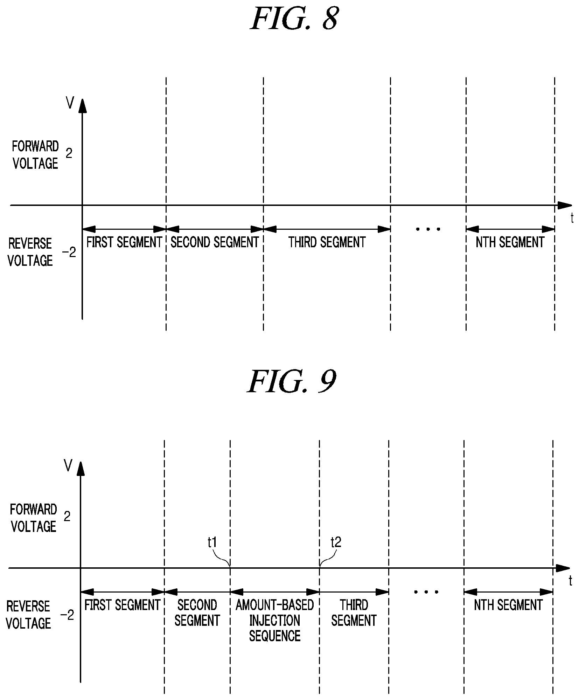

A pair of pulse signals 21 and 22 included in the pulse block 20 may be voltage pulse signals or current pulse signals. A pair of voltage pulse signals may be composed of a forward voltage pulse and a reverse voltage pulse, and a pair of current pulse signals may be composed of a forward current pulse and a reverse current pulse. Herein, the pair of voltage pulse signals may include information about amplitudes and durations of the respective voltage pulses, and the pair of current pulse signals may include information about amplitudes and durations of the respective current pulses. For example, in the speed-based injection sequence illustrated in **FIG. 8****,** the amplitude of a pulse signal may be 2 V, and the duration thereof may be 10 s.

Each pulse block 20 included in the speed-based injection sequence may include voltage pulses having different voltage levels or current pulses having different current levels. Alternatively, each pulse block 20 may include voltage pulses having the same voltage level or current pulses having the same current level, but respective durations may be set differently.

Further, the forward pulse 21 and the reverse pulse 22 included in the pulse block 20 may be set to have the same amplitude and the same duration. Accordingly, the amounts of drug sucked and discharged by the electroosmotic pump 110 may remain the same.

Furthermore, the pair of pulse signals 21 and 22 included in the pulse block 20 may be provided as a constant voltage or a constant current, and the amounts of drug sucked and discharged by each pulse block may be regulated by controlling the duration of a pulse signal provided by a constant voltage or a constant current. For example, the pulse blocks 20 of **FIG. 8** are provided by a constant voltage of 2 V.

Also, the amplitudes and durations of the pair of pulse signals 21 and 22 may be controlled. Accordingly, the amounts of drug sucked and discharged may be regulated to be the same. For example, the amplitude of a forward voltage pulse may be 2 V and the duration thereof may be 10 s, and the amplitude of a subsequent reverse voltage pulse may be set to 1 V and the duration thereof may be set to 20 s. Accordingly, the area of the forward pulse and the area of the reverse pulse are set to be the same to ensure that the amounts of drug sucked and discharged remain the same.

Each pulse block 20 may further include a stabilization pulse 23, and the stabilization pulse 23 is a pulse that maintains a voltage of 0 V for a predetermined period of time after the forward voltage pulse 21 and the reverse voltage pulse 22 are applied. An operation of the electroosmotic pump 110 may be stabilized by the stabilization pulse 23. Herein, when the pulse block 10 of a speed-based injection sequence is composed of a pair of current pulses, the pulse block 20 may further include the stabilization pulse 23 that maintains a current of 0 A for a predetermined period of time after the forward current pulse and the reverse current pulse are applied.

The pause block 30 is a signal that is provided after the pulse block 20 is applied and maintains a voltage of 0 V or a current of 0 A for a predetermined period of time. The drug injector 100 of the present disclosure injects the drug in an amount equivalent to the drug injection amount for a predetermined period of time. Therefore, as the speed-based injection sequence includes the pause block 30, it is possible to control the time at which a target injection amount of drug is injected, and by minimizing a time period during which the drug is not injected, it is possible to suppress blockage in a flow path of the drug injector 100 during the time period during which the drug is not injected.

The controller 200 generates a control signal including a voltage pulse or a current pulse corresponding to the speed-based injection sequence based on information of the pulse block 20 included in the speed-based injection sequence, and applies the control signal to the drug injector 100.

When the control signal corresponding to the speed-based injection sequence is applied to the electroosmotic pump 110, the electroosmotic pump 110 alternately generates negative pressure and positive pressure for each pulse block to suck and discharge the drug. Herein, the control signal may be a signal composed of a pair of voltage pulses including a forward voltage pulse and a reverse voltage pulse in units of pulse blocks 20 or a pair of current pulses including a forward current pulse and a reverse current pulse in units of pulse blocks 20.

**FIG. 8** is an example diagram illustrating a signal structure for a first speed-based injection sequence for a basal injection mode according to an embodiment of the present disclosure, and **FIG. 9** is an example diagram illustrating a signal structure in which a second speed-based injection sequence for a temporary basal injection mode is applied to the first speed-based injection sequence illustrated in **FIG. 8****.**

Referring to **FIG. 8** and **FIG. 9****,** an example where a temporary basal injection mode is applied to the basal injection mode will be described. In the basal injection mode shown in **FIG. 8****,** 24 hours are divided into a plurality of segments, each assigned a specific duration and drug injection speed. The drug injector 100 performs the basal injection mode by executing the segments in the order shown in **FIG. 8****.** When the temporary basal injection mode is executed at a time point t1 while the drug injector 100 executes a first segment, the second speed-based injection sequence for the temporary basal injection mode is arranged midway through the second segment as shown in **FIG. 9** and executed accordingly. When the temporary basal injection mode ends at a time pint t2, the controller 200 transmits the first speed-based injection sequence for the basal injection mode, corresponding to the time point t2, to the drug injector 100, which then resumes the basal injection mode. Herein, a portion of the first speed-based injection sequence for the basal injection mode between the time point t1 and the time point t2 is omitted due to the execution of the second speed-based injection sequence for the temporary basal injection mode.

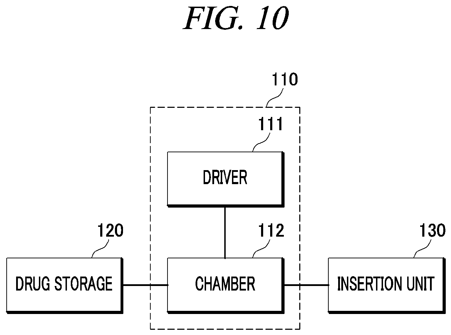

**FIG. 10** is a block diagram schematically illustrating a configuration of an electroosmotic pump illustrated in **FIG. 2****.**

The electroosmotic pump 110 will be described in detail with reference to **FIG. 10****.** The electroosmotic pump 110 includes a driver 111 and a chamber 112. The driver 111 is electrochemically driven in response to a control signal to generate positive pressure and negative pressure and discharges a drug according to the positive pressure and the negative pressure, and the chamber 112 sucks the drug from a drug storage 120 according to the pressure of the driver 111 and then discharges the drug to an insertion unit 130. Herein, the drug needs to be injected into a specific patient and may be, for example, insulin that is injected into a diabetic patient.

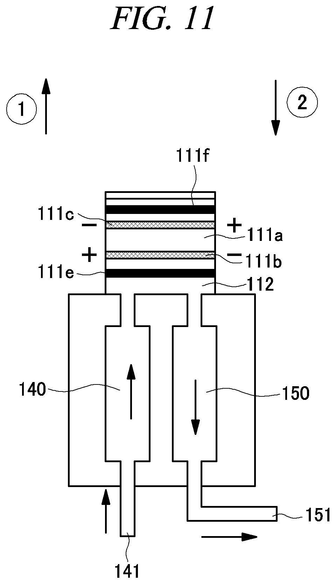

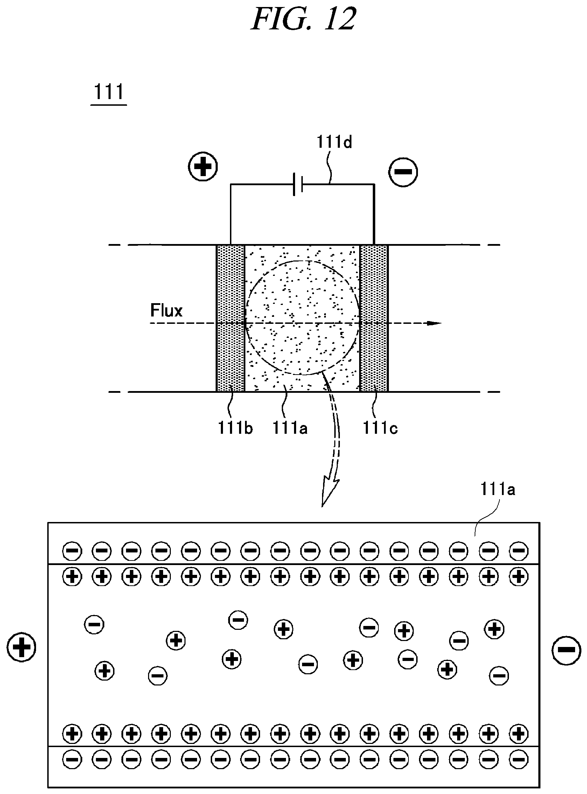

**FIG. 11** is a conceptual diagram schematically illustrating a configuration of a driver illustrated in **FIG. 10****,** and **FIG. 12** is an example diagram schematically illustrating an operation of the driver illustrated in **FIG. 11****.**

The driver 111 will be described in detail with reference to **FIG. 11** and **FIG. 12****.** The driver 111 is a pump that uses the movement of a fluid according to electroosmosis that occurs when a voltage or current is applied by using electrodes at both ends of a porous film (membrane) 111a. The driver 111 may include the membrane 111a, a power source 111d that applies a voltage or current to a first electrode 111b and a second electrode 111c arranged on both sides of the membrane 111a, and a flow path through which a fluid moves.

Also, the driver 111 may include a first diaphragm 111e arranged adjacent to the first electrode 111b and a second diaphragm 111f arranged adjacent to the second electrode 111c. The first and second diaphragms 111e and 111f are provided respectively one side and the other side of the membrane 111a, and deformed in shape by the movement of a pumping solution as positive pressure and negative pressure are alternately generated. For example, the first diaphragm 111e and the second diaphragm 111f transfer the negative pressure and positive pressure generated by an operation of the membrane 111a to a transfer target fluid. More specifically, when negative pressure is generated, at least a part of the first diaphragm 111e and the second diaphragm 111f moves backwards (*i.e.*, moves in a direction ①) such that the transfer target fluid is sucked to the chamber 112, and when positive pressure is generated, at least a part of the first diaphragm 111e and the second diaphragm 111f moves forwards (*i.e.*, moves in a direction ②) such that the transfer target fluid is discharged from the chamber 112.

Silica, glass, and the like are generally used as a material of the porous film 111a, and when the silica and glass are immersed in an aqueous solution, surfaces thereof are negatively charged. There are many paths, through which fluids may pass, in the porous film 111a, and when one of the paths is enlarged, a surface of the fluid path with negative charges (bound anions) may be in a state in which the negative charges are balanced by mobile cations having positive charges. In this state, when a positive voltage is applied to the first electrode 111b and a negative voltage is applied to the second electrode 111c, negative pressure is generated, and the fluid inside the driver 111 moves in the direction ① due to the negative pressure. In this case, a drug is sucked through an inflow path 141 and flows into the chamber 112 through an inflow valve 140. In this case, an outflow valve 150 is closed such that the negative pressure is not transferred to an outflow path 151. When a negative voltage is applied to the first electrode 111b and a positive voltage is applied to the second electrode 111c, the positive pressure in an opposite direction is generated by a reversible electrochemical reaction. The fluid inside the driver 111 moves in the direction ② by the positive pressure. In this case, the drug stored in the chamber 112 is injected into a target through the outflow valve 150 and the outflow path 151. In this case, the inflow valve 140 is closed such that the positive pressure is not transferred to the inflow path 141.

This phenomenon is called electroosmosis, and a pump that uses this principle is the electroosmotic pump 110.

An electrode used for the driver 111 may be implemented by a platinum (Pt) mesh, such as a porous electrode, porous carbon paper or carbon cloth, or various electrode materials applied onto a porous structure so as to smoothly move a fluid.

Also, the electrode used for the driver 111 may be coated with various fixable materials onto an impermeable substrate material by drop coating or spin coating. In this case, the impermeable substrate material is a plate-shaped substrate including at least one of a conductive material, a semiconductor material, and a non-conductive material, and an electrode material coated thereon may include metal, metal oxide, a conducting polymer, metal hexacyanoferrate, a carbon nanostructure, or a composite thereof.

When polarities of a voltage or current are alternately supplied to the first electrode 111b and the second electrode 111c of the driver 111, reversible electrochemical reactions occur in forward and reverse directions. As the forward and reverse electrochemical reactions occur repeatedly, the fluid inside the electroosmotic pump 110 repeatedly reciprocates back and forth. Further, the electrode materials of the first electrode 111b and the second electrode 111c are repeatedly consumed and regenerated by the repetitive reversible electrochemical reactions in the forward and reverse directions. If the inflow valve 140 and the outflow valve 150 are coupled to the chamber 112 of the electroosmotic pump 110, the drug in the drug storage 120 is sucked through the inflow path 141 and stored in the chamber 112 through the inflow valve 140 during the inflow operation. Furthermore, the drug stored in the chamber 112 is discharged to the insertion unit 130 through the outflow valve 150 and the outflow path 151 during the outflow operation.

Therefore, the pressure generated by the electroosmotic pump 110 and the volume of the drug discharged from the electroosmotic pump 110 can be controlled by controlling the amplitudes and application times of voltages or currents applied to the first and second electrodes 111b and 111c.

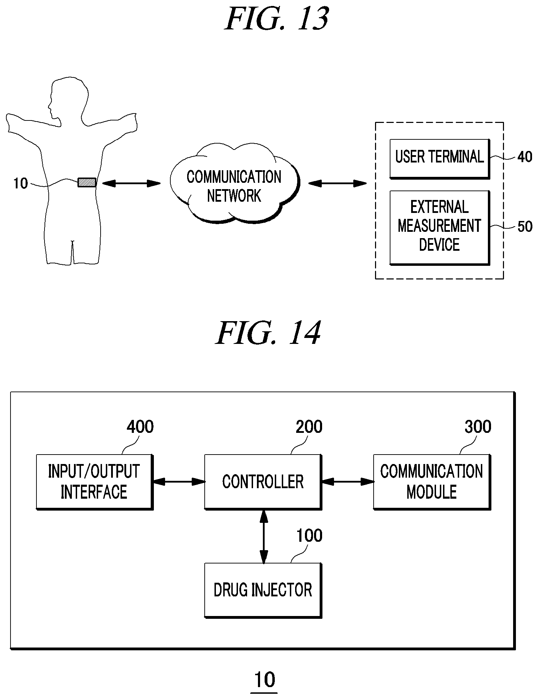

**FIG. 13** illustrates an example of application of the drug injection device according to an embodiment of the present disclosure, and **FIG. 14** is a block diagram schematically illustrating a configuration of the drug injection device illustrated in **FIG. 13****.** An operation of the drug injection device 10 will be described in detail with reference to **FIG. 13** and **FIG. 14****.**

The drug injection device 10, which is operating based on the first speed-based injection sequence for the basal injection mode, receives predetermined information from the user or an external device and sets the second speed-based injection sequence for the temporary basal injection mode based on the information. A process of setting the second speed-based injection sequence may be performed according to data used to set the second speed-based injection sequence. Hereafter, the process of setting the second speed-based injection sequence for the temporary basal injection mode will be described.

The drug injection device 10 may detect an event requesting the activation of the temporary basal injection mode and set the second speed-based injection sequence accordingly. To detect such an event, the drug injection device 10 may further include a communication module 300 and a user input/output interface 400.

When an event requesting the activation of the temporary basal injection mode is detected via the user device 40 communicatively connected to the communication module 300 or via the user input/output interface 400 executed by the controller 200, the drug injection device 10 allows the user to input an adjustment ratio and an adjustment period of the first speed-based injection sequence for the basal injection mode currently being executed or to input a drug injection period and a drug injection speed as temporary basal injection conditions via the user device 40 or the user input/output interface 400. Also, the drug injection device 10 sets, as the second speed-based injection sequence, a speed-based injection sequence based on the input adjustment ratio and adjustment period or a speed-based injection sequence corresponding to the input drug injection period and drug injection speed. Herein, the adjustment ratio refers to a ratio of adjusting the injection amounts for the segments included in the first speed-based injection sequence. When the adjustment ratio is 50%, the injection amount is reduced by half. Also, when the adjustment ratio is 120%, the injection amount is increased by 20%. Further, the adjustment period refers to a period during which the adjustment ratio is applied to the first speed-based injection sequence.

The user device 40 connected to the drug injection device 10 may be implemented by a computer or portable device that can be connected to the drug injection device 10 through wireless communication. Herein, the computer includes, for example, a desktop computer, a laptop computer, and the like equipped with a web browser, and the portable device is, for example, a wireless communication device that ensures portability and mobility and may include all kinds of handheld wireless communication devices, such as various smartphones, tablet personal computers (PCs), and smart watches. The user device 40 is managed by a wearer of the drug injection device 10 or a medical professional, and drug injection conditions may be set through an application executed on the user device 40.

The user input/output interface module 400 may include a physical input/output button coupled to an external housing including the drug injection device 10, and a signal processing circuit that transmits a signal generated according to an operation of the physical input/output button to the controller 200. Alternatively, the user input/output interface module 400 may output a user interface (UI) that guides the user to input information about the drug injection conditions or speed-based injection sequence through a display in the form of a touch screen.

Subsequently, the drug injection device 10 may set the second speed-based injection sequence based on the user's activity level data. Herein, the user's activity level data may include the user's heart rate or step count. When the user's activity level data collected through a program executed on the controller 200 or an external measurement device 50 communicatively connected to the communication module 300 exceeds a first threshold or falls below a second threshold, the drug injection device 10 outputs an event. Based on this event, the drug injection device 10 outputs the user input/output interface 400 to allow the user to input temporary basal injection conditions. Thus, the drug injection device 10 allows the user to input an adjustment ratio and an adjustment period of the first speed-based injection sequence for the basal injection mode currently being executed or to input a drug injection period and a drug injection speed as the temporary basal injection conditions. Also, the drug injection device 10 sets, as the second speed-based injection sequence, a speed-based injection sequence based on the input adjustment ratio and adjustment period or a speed-based injection sequence corresponding to the input drug injection period and drug injection speed. Herein, the external measurement device 50 may be a wearable device to be worn on a portion of the user's body.

Further, the drug injection device 10 may set the second speed-based injection sequence based on the user's blood sugar level. When the user's blood sugar level collected through the program executed on the controller 200 or the external measurement device 50 communicatively connected to the communication module 300 is out of a first range or maintains a second range for a predetermined period of time, the drug injection device 10 outputs an event. Based on this event, the drug injection device 10 outputs the user input/output interface 400 to allow the user to input temporary basal injection conditions. Thus, the drug injection device 10 allows the user to input an adjustment ratio and an adjustment period of the first speed-based injection sequence for the basal injection mode currently being executed or to input a drug injection period and a drug injection speed as the temporary basal injection conditions. Also, the drug injection device 10 sets, as the second speed-based injection sequence, a speed-based injection sequence based on the input adjustment ratio and adjustment period or a speed-based injection sequence corresponding to the input drug injection period and drug injection speed.

Furthermore, in the process of setting the second speed-based injection sequence for temporary basal injection, the controller 200 may set, as the second speed-based injection sequence, a speed-based injection sequence based on an adjustment ratio and an adjustment period for the first speed-based injection sequence or a speed-based injection sequence corresponding to a drug injection period and a drug injection speed with reference to a table that stores a plurality of speed-based injection sequences.

When the second speed-based injection sequence for temporary basal injection is set based on an adjustment ratio and an adjustment period, if the adjustment period includes a plurality of segments, the adjustment ratio can be applied to each segment to regulate the drug injection amount.

For example, referring to **FIG. 7** and **FIG. 9****,** the adjustment period represents the length of the second speed-based injection sequence and the adjustment ratio is assumed to be 50%. If this is applied to **FIG. 9**, the adjustment period includes a portion of the second segment in which the drug is injected at 0.4 U/h and a portion of the third segment in which the drug is injected at 0.8 U/h. When the adjustment ratio of 50% is applied to each segment, the drug is injected at 0.2 U/h in the portion of the second segment included in the adjustment period and at 0.4 U/h in the portion of the third segment.

Also, one of the plurality of segments may be selected, and the adjustment ratio may be applied to the selected segment and assigned to the entire adjustment period.

For example, referring to **FIG. 7** and **FIG. 9****,** if the second segment is selected from among the second and third segments, the drug is injected at 0.2 U/h for the entire adjustment period.

When the execution of the temporary basal injection mode using the second speed-based injection sequence is completed, the drug injection device 10 receives the user's activity level data or blood sugar level information from the program executed on the controller 200 or one or more external measurement devices 50 communicatively connected to the communication module 300. Based on the user's activity level data or blood sugar level information, the drug injection device 10 determines whether to continue the temporary basal injection mode. Herein, the user's activity level data includes the user's heart rate or step count.

Whether to continue the temporary basal injection mode is determined based on the same conditions that were applied to the process of setting the second speed-based injection sequence based on the user's activity level data or blood sugar level information. If it is determined that the temporary basal injection mode is further needed, the process of setting the second speed-based injection sequence can be performed again.

However, if it is not determined that the temporary basal injection mode is further needed, the controller 200 resumes outputting the control signal for the first speed-based injection sequence that was temporarily stopped, but omits an operation according to the first speed-based injection sequence which is scheduled to be performed during the pause period and performs an operation according to the first speed-based injection sequence which is scheduled to be performed immediately after the operation based on the temporary basal injection mode is completed.

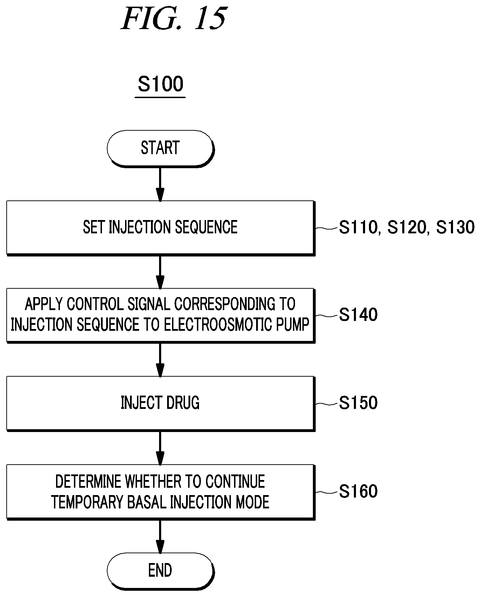

Hereafter, a drug injection method performed by the drug injection device will be described with reference to **FIG. 15**.

**FIG. 15** is a flowchart illustrating a drug injection method according to an embodiment of the present disclosure.

Referring to **FIG. 1, FIG. 2** and **FIG. 15****,** in a drug injection method S100 according to an embodiment of the present disclosure, the controller 200 sets a speed-based injection sequence determined according to an injection mode when a predetermined event occurs (processes S110, S120 and S130), and applies a control signal corresponding to the speed-based injection sequence to the electroosmotic pump 110 (process S140). Thereafter, in response to the control signal, the electroosmotic pump 110 alternately generates negative pressure and positive pressure for each pulse block to suck and discharge a drug and inject the drug into a user (process S150). In the process S150, the controller 200 stops an operation based on a first speed-based injection sequence for a basal injection mode and outputs, to the electroosmotic pump 110, a control signal corresponding to a second speed-based injection sequence for a temporary basal injection mode. Herein, the first and second speed-based injection sequences are injection sequences set based on a drug injection speed.

Hereafter, each process will be described in detail.

In the process of setting the speed-based injection sequence (process S110), the drug injection device 10, which is operating based on the first speed-based injection sequence for the basal injection mode, receives predetermined information from the user or an external device and sets the second speed-based injection sequence for the temporary basal injection mode based on the information. The process of setting the second speed-based injection sequence may be performed according to data used to set the second speed-based injection sequence.

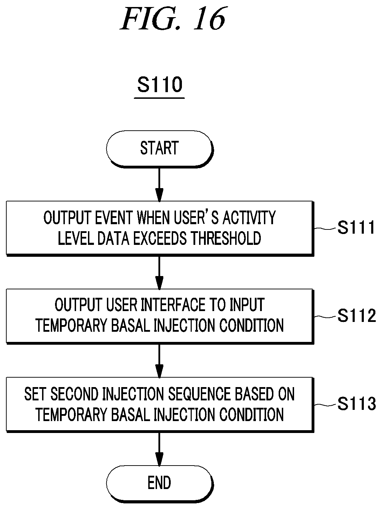

**FIG. 16** is a flowchart for describing a process of setting a second speed-based injection sequence according to an embodiment of the present disclosure.

The process of setting the second speed-based injection sequence for the temporary basal injection mode by the drug injection device 10 according to an embodiment of the present disclosure will be described with reference to **FIG. 14** and **FIG. 16****.**

According to the present embodiment, the drug injection device 10 may detect an event requesting the activation of the temporary basal injection mode and set the second speed-based injection sequence accordingly. Specifically, when an event requesting the activation of the temporary basal injection mode is detected via the user device 40 communicatively connected to the communication module 300 or via the user input/output interface 400 executed by the controller 200 (process S111), the drug injection device 10 allows the user to input an adjustment ratio and an adjustment period of the first speed-based injection sequence for the basal injection mode currently being executed or to input a drug injection period and a drug injection speed as temporary basal injection conditions via the user device 40 or the user input/output interface 400 (process S112). Also, the drug injection device 10 sets, as the second speed-based injection sequence, a speed-based injection sequence based on the input adjustment ratio and adjustment period or a speed-based injection sequence corresponding to the input drug injection period and drug injection speed (process S113).

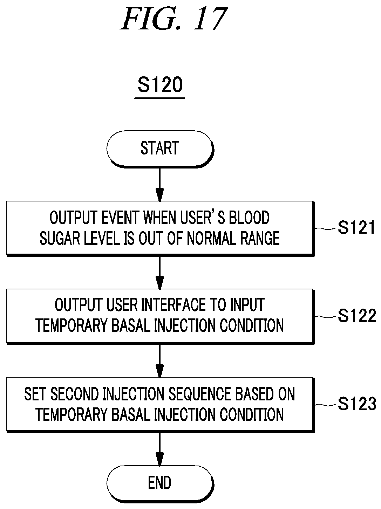

**FIG. 17** is a flowchart for describing the process of setting the second speed-based injection sequence according to another embodiment of the present disclosure.

The process of setting the second speed-based injection sequence for the temporary basal injection mode by the drug injection device 10 according to an embodiment of the present disclosure will be described with reference to **FIG. 14** and **FIG. 17****.**

According to the present embodiment, the drug injection device 10 may set the second speed-based injection sequence based on the user's activity level data. The user's activity level data may include the user's heart rate or step count. Specifically, when the user's activity level data collected through the program executed on the controller 200 or the external measurement device 50 communicatively connected to the communication module 300 exceeds a first threshold or falls below a second threshold, the drug injection device 10 outputs an event (process S121), and based on this event, the drug injection device 10 outputs the user input/output interface 400 to allow the user to input temporary basal injection conditions. Thus, the drug injection device 10 allows the user to input an adjustment ratio and an adjustment period of the first speed-based injection sequence for the basal injection mode currently being executed or to input a drug injection period and a drug injection speed as the temporary basal injection conditions (process S122). Also, the drug injection device 10 sets, as the second speed-based injection sequence, a speed-based injection sequence based on the input adjustment ratio and adjustment period or a speed-based injection sequence corresponding to the input drug injection period and drug injection speed (process S123). Herein, the external measurement device 50 may be a wearable device to be worn on a portion of the user's body.

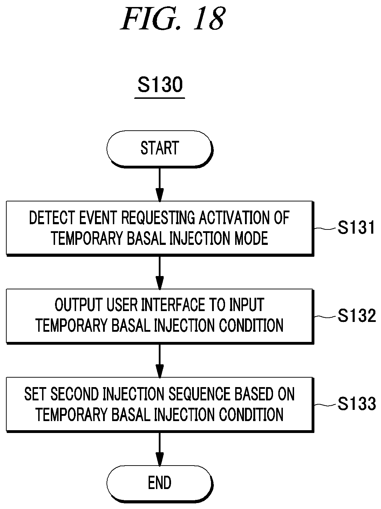

**FIG. 18** is a flowchart for describing the process of setting the second speed-based injection sequence according to yet another embodiment of the present disclosure.

The process of setting the second speed-based injection sequence for the temporary basal injection mode by the drug injection device 10 according to an embodiment of the present disclosure will be described with reference to **FIG. 14** and **FIG. 18****.**

According to the present embodiment, the drug injection device 10 may set the second speed-based injection sequence based on the user's blood sugar level information. Specifically, when the user's blood sugar level collected through the program executed on the controller 200 or the external measurement device 50 communicatively connected to the communication module 300 is out of a first range or maintains a second range for a predetermined period of time, the drug injection device 10 outputs an event (process S131), and based on this event, the drug injection device 10 outputs the user input/output interface 400 to allow the user to input temporary basal injection conditions. Thus, the drug injection device 10 allows the user to input an adjustment ratio and an adjustment period of the first speed-based injection sequence for the basal injection mode currently being executed or to input a drug injection period and a drug injection speed as the temporary basal injection conditions (process S132). Also, the drug injection device 10 sets, as the second speed-based injection sequence, a speed-based injection sequence based on the input adjustment ratio and adjustment period or a speed-based injection sequence corresponding to the input drug injection period and drug injection speed (process S133).

In the above-described embodiments of setting the second speed-based injection sequence for temporary basal injection (processes S110, S120 and S130), the controller 200 may set, as the second speed-based injection sequence, a speed-based injection sequence corresponding to an adjustment ratio and an adjustment period for the first speed-based injection sequence or a speed-based injection sequence corresponding to a drug injection period and a drug injection speed with reference to a table that stores a plurality of speed-based injection sequences.

In the process of setting the second speed-based injection sequence based on an adjustment ratio and an adjustment period, if the adjustment period includes a plurality of segments, the adjustment ratio can be applied to each segment to regulate the drug injection amount.

For example, referring to **FIG. 7** and **FIG. 9****,** the adjustment period represents the length of the second speed-based injection sequence and the adjustment ratio is assumed to be 50%. If this is applied to **FIG. 9****,** the adjustment period includes a portion of the second segment in which the drug is injected at 0.8 U/h and a portion of the third segment in which the drug is injected at 0.4 U/h. When the adjustment ratio of 50% is applied to each segment, the drug is injected at 0.4 U/h in the portion of the second segment included in the adjustment period and at 0.2 U/h in the portion of the third segment.

Also, one of the plurality of segments may be selected, and the adjustment ratio may be applied to the selected segment and assigned to the entire adjustment period.

For example, referring to **FIG. 7** and **FIG. 9****,** if the second segment is selected from among the second and third segments included in the adjustment period, the drug is injected at 0.4 U/h for the entire adjustment period.

When the injection based on the drug in the temporary basal injection mode is completed through the process S150, the drug injection device 10 receives the user's activity level data or blood sugar level information from the program executed on the controller 200 or one or more external measurement devices 50 communicatively connected to the communication module 300. Based on the user's activity level data or blood sugar level information, the drug injection device 10 may further perform a process of determining whether to continue the temporary basal injection mode (process S160). Herein, the user's activity level data includes the user's heart rate or step count.

Whether to continue the temporary basal injection mode is determined based on the same conditions that were applied to the process of setting the second speed-based injection sequence based on the user's activity level data or blood sugar level information. If it is determined that the temporary basal injection mode is further needed, the process of setting the second speed-based injection sequence (processes S120 and S130) can be performed again.

However, if it is not determined that the temporary basal injection mode is further needed, the controller 200 resumes outputting the control signal for the first speed-based injection sequence that was temporarily stopped, but omits an operation according to the first speed-based injection sequence which is scheduled to be performed during the pause period and performs an operation according to the first speed-based injection sequence which is scheduled to be performed immediately after the operation based on the temporary basal injection mode is completed.

The method according to an embodiment of the present disclosure can be embodied in a storage medium including instruction codes executable by a computer such as a program module executed by the computer. A computer-readable medium can be any usable medium which can be accessed by the computer and includes all volatile/non-volatile and removable/non-removable media. Further, the computer-readable medium may include all computer storage media. The computer storage media include all volatile/non-volatile and removable/non-removable media embodied by a certain method or technology for storing information such as computer-readable instruction code, a data structure, a program module or other data.

The method and system of the present disclosure have been explained in relation to a specific embodiment, but their components or a part or all of their operations can be embodied by using a computer system having general-purpose hardware architecture.

It would be understood by a person with ordinary skill in the art that various changes and modifications may be made based on the above description without changing technical conception and essential features of the present disclosure. Thus, it is clear that the above-described embodiments are illustrative in all aspects and do not limit the present disclosure. The scope of the present disclosure is defined by the following claims. It shall be understood that all modifications and embodiments conceived from the meaning and scope of the claims and their equivalents are included in the scope of the present disclosure.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. A drug injection device, comprising:
a drug injector configured to electrochemically drive an electroosmotic pump to suck a drug from a drug storage and discharge the sucked drug to an injection target; and
a controller configured to output, to the drug injector, a control signal corresponding to a speed-based injection sequence determined according to an injection mode,
wherein when a predetermined event occurs, the controller stops an operation based on a first speed-based injection sequence for a basal injection mode and outputs a control signal corresponding to a second speed-based injection sequence for a temporary basal injection mode, and
the electroosmotic pump alternately generates negative pressure and positive pressure in response to the control signal to suck and discharge the drug.

2. The drug injection device of Claim 1,
wherein the speed-based injection sequence includes at least one pulse block that defines a voltage pulse or a current pulse to be applied to the electroosmotic pump and at least one pause block that maintains a voltage of 0 V or a current of 0 A for a predetermined period of time after the pulse block is applied,
each pulse block defines a pair of pulse signals including a forward pulse and a reverse pulse that are applied to the electroosmotic pump to alternately generate negative pressure and positive pressure, and
the electroosmotic pump alternately generates negative pressure and positive pressure for each pulse block to suck and discharge the drug.

3. The drug injection device of Claim 2,
wherein the speed-based injection sequence is configured by arranging a plurality of pulse blocks that satisfies a drug injection speed based on a drug injection period and the drug injection speed that define the basal injection mode or the temporary basal injection mode, and
the sum of durations of the plurality of pulse blocks and a plurality of pause blocks following the pulse blocks is set to correspond to the drug injection period.

4. The drug injection device of Claim 1,
wherein the controller outputs the event when a user's activity level data collected through a program executed on the controller or one or more wearable devices communicatively connected to the drug injection device exceeds a first threshold or falls below a second threshold,
based on this event, the controller outputs a user interface to allow the user to input temporary basal injection conditions and thus allows the user to input an adjustment ratio and an adjustment period of the first speed-based injection sequence or to input a drug injection period and a drug injection speed as the temporary basal injection conditions, and
the controller sets, as the second speed-based injection sequence, a speed-based injection sequence based on the input adjustment ratio and adjustment period or a speed-based injection sequence corresponding to the input drug injection period and drug injection speed.

5. The drug injection device of Claim 4,
wherein the user's activity level data includes the user's heart rate or step count.

6. The drug injection device of Claim 1,
wherein the controller outputs the event when a user's blood sugar level collected through a program executed on the controller or one or more wearable devices communicatively connected to the drug injection device is out of a first range or maintains a second range for a predetermined period of time,
based on this event, the controller outputs a user interface to allow the user to input temporary basal injection conditions and thus allows the user to input an adjustment ratio and an adjustment period of the first speed-based injection sequence or to input a drug injection period and a drug injection speed as the temporary basal injection conditions, and
the controller sets, as the second speed-based injection sequence, a speed-based injection sequence based on the input adjustment ratio and adjustment period or a speed-based injection sequence corresponding to the input drug injection period and drug injection speed.

7. The drug injection device of Claim 1,
wherein when an event requesting activation of temporary basal injection is detected via a user interface executed by the controller, the controller allows a user to input an adjustment ratio and an adjustment period of the first speed-based injection sequence or to input a drug injection period and a drug injection speed as temporary basal injection conditions via the user interface, and
the controller sets, as the second speed-based injection sequence, a speed-based injection sequence based on the input adjustment ratio and adjustment period or a speed-based injection sequence corresponding to the input drug injection period and drug injection speed.

8. The drug injection device of any one of Claim 4, Claim 6, and Claim 7,
wherein the speed-based injection sequence is configured by arranging a pulse block and a pause block assigned to each of a plurality of segments into which the drug injection period is divided in predetermined time units in order for the drug injection speed to be the same for some segments and to be different for some segments.

9. The drug injection device of Claim 8,
wherein if the plurality of segments is included in the adjustment period, the controller applies the adjustment ratio to each segment.

10. The drug injection device of Claim 8,
wherein if the plurality of segments is included in the adjustment period, one of the plurality of segments is selected and the controller applies the adjustment ratio to the selected segment and assigns the adjustment ratio to the entire adjustment period.

11. The drug injection device of Claim 1,
wherein when an operation based on the temporary basal injection mode is completed, the controller resumes outputting a control signal for the first speed-based injection sequence that was temporarily stopped.

12. The drug injection device of Claim 1,
wherein when an operation based on the temporary basal injection mode is completed, the controller resumes outputting a control signal for the first speed-based injection sequence that was temporarily stopped, and
the controller omits an operation according to the first speed-based injection sequence which is scheduled to be performed during the pause period and performs an operation according to the first speed-based injection sequence which is scheduled to be performed immediately after the operation based on the temporary basal injection mode is completed.

13. The drug injection device of Claim 1,
wherein when execution of the temporary basal injection mode is completed, the controller receives a user's activity level data or blood sugar level information from a program executed on the controller or one or more wearable devices communicatively connected to the drug injection device, and
the controller determines whether to continue the temporary basal injection mode based on the user's activity level data or blood sugar level information, and
the user's activity level data includes the user's heart rate or step count.

14. The drug injection device of Claim 3,
wherein the controller sets, as the second speed-based injection sequence, a speed-based injection sequence corresponding to temporary basal injection conditions with reference to a table that stores a plurality of speed-based injection sequences according to the temporary basal injection conditions.

15. The drug injection device of Claim 3,
wherein the speed-based injection sequence is configured by arranging M number of pulse blocks (M is a natural number less than or equal to N) selected from among N number of pulse blocks (N is a natural number), which supply different amounts of drug, based on temporary basal injection conditions, and
the sum of durations of the M number of selected pulse blocks and a plurality of pause blocks following the pulse blocks is set to correspond to the drug injection period.

16. The drug injection device of Claim 15,
wherein the M number of pulse blocks of the speed-based injection sequence are set to satisfy the drug injection speed of the temporary basal injection conditions while ensuring that the total number of times the selected pulse blocks are used matches a predetermined number or a maximum number and a duration of the pause block following each pulse block is less than a recommended pause period.

17. The drug injection device of Claim 2,
wherein the speed-based injection sequence is configured by arranging a plurality of pulse blocks and pause blocks for a unit time, and
the controller repeatedly supplies the speed-based injection sequence during the drug injection period.

18. The drug injection device of Claim 2,
wherein a duration of each pause block in the speed-based injection sequence is less than a recommended pause period.

19. The drug injection device of Claim 2,
wherein in the speed-based injection sequence,
a pulse block supplying the largest amount of drug among a plurality of pulse blocks is output first.

20. The drug injection device of Claim 2,
wherein the pair of pulse signals are composed of a pair of voltage pulses including a forward voltage pulse and a reverse voltage pulse, and include a stabilization pulse that maintains a voltage of 0 V for a predetermined period of time after the forward voltage pulse and the reverse voltage pulse are applied, or
the pair of pulse signals are composed of a pair of current pulses including a forward current pulse and a reverse current pulse, and include a stabilization pulse that maintains a current of 0 A for a predetermined period of time after the forward current pulse and the reverse current pulse are applied.

21. The drug injection device of Claim 1, further comprising:
a driver that is electrochemically driven in response to the control signal to alternately generate positive pressure and negative pressure; and
a chamber that sucks the drug from the drug storage according to a pressure of the driver and then discharges the drug to an insertion unit.

22. A drug injection method performed by a drug injection device including an electroosmotic pump, comprising:
setting a speed-based injection sequence determined according to an injection mode when a predetermined event occurs;
applying a control signal corresponding to the speed-based injection sequence to the electroosmotic pump; and
causing the electroosmotic pump to alternately generate negative pressure and positive pressure for each pulse block in response to the control signal to suck and discharge a drug,
wherein the process of applying the control signal to the electroosmotic pump includes stopping an operation based on a first speed-based injection sequence for a basal injection mode and outputting a control signal corresponding to a second speed-based injection sequence for a temporary basal injection mode.

23. The drug injection method of Claim 22,
wherein the speed-based injection sequence includes at least one pulse block that defines a voltage pulse or a current pulse to be applied to the electroosmotic pump and at least one pause block that maintains a voltage of 0 V or a current of 0 A for a predetermined period of time after the pulse block is applied,
each pulse block defines a pair of pulse signals including a forward pulse and a reverse pulse that are applied to the electroosmotic pump to alternately generate negative pressure and positive pressure, and
the electroosmotic pump alternately generates negative pressure and positive pressure for each pulse block to suck and discharge the drug.

24. The drug injection method of Claim 23,
wherein the speed-based injection sequence is configured by arranging a plurality of pulse blocks that satisfies a drug injection speed based on a drug injection period and the drug injection speed that define the basal injection mode or the temporary basal injection mode, and
the sum of durations of the plurality of pulse blocks and a plurality of pause blocks following the pulse blocks is set to correspond to the drug injection period.

25. The drug injection method of Claim 22,
wherein the process of setting the speed-based injection sequence includes:
outputting the event when a user's activity level data collected through a program executed on the drug injection device or one or more wearable devices communicatively connected to the drug injection device exceeds a first threshold or falls below a second threshold;
outputting a user interface based on this event to allow the user to input temporary basal injection conditions and thus allows the user to input an adjustment ratio and an adjustment period of the first speed-based injection sequence or to input a drug injection period and a drug injection speed as the temporary basal injection conditions; and
setting, as the second speed-based injection sequence, a speed-based injection sequence based on the input adjustment ratio and adjustment period or a speed-based injection sequence corresponding to the input drug injection period and drug injection speed.

26. The drug injection method of Claim 25,
wherein the user's activity level data includes the user's heart rate or step count.

27. The drug injection method of Claim 22,
wherein the process of setting the speed-based injection sequence includes:
outputting the event when a user's blood sugar level collected through a program executed on the drug injection device or one or more wearable devices communicatively connected to the drug injection device is out of a first range or maintains a second range for a predetermined period of time;
outputting a user interface based on this event to allow the user to input temporary basal injection conditions and thus allows the user to input an adjustment ratio and an adjustment period of the first speed-based injection sequence or to input a drug injection period and a drug injection speed as the temporary basal injection conditions; and
setting, as the second speed-based injection sequence, a speed-based injection sequence based on the input adjustment ratio and adjustment period or a speed-based injection sequence corresponding to the input drug injection period and drug injection speed.

28. The drug injection method of Claim 22,
wherein the process of setting the speed-based injection sequence includes:
detecting an event requesting activation of a temporary basal injection mode via a user interface executed by the drug injection device;
allowing a user to input an adjustment ratio and an adjustment period of the first speed-based injection sequence or to input a drug injection period and a drug injection speed as temporary basal injection conditions via the user interface; and
setting, as the second speed-based injection sequence, a speed-based injection sequence based on the input adjustment ratio and adjustment period or a speed-based injection sequence corresponding to the input drug injection period and drug injection speed.

29. The drug injection method of any one of Claim 25, Claim 27, and Claim 28,
wherein the speed-based injection sequence is configured by arranging a pulse block and a pause block assigned to each of a plurality of segments into which the drug injection period is divided in predetermined time units in order for the drug injection speed to be the same for some segments and to be different for some segments.

30. The drug injection method of Claim 29,
wherein in the process of setting the second speed-based injection sequence, if the plurality of segments is included in the adjustment period, one of the plurality of segments is selected and the adjustment ratio is applied to the selected segment and assigned to the entire adjustment period.

31. The drug injection method of Claim 29,
wherein in the process of setting the second speed-based injection sequence, if the plurality of segments is included in the adjustment period, the adjustment ratio is applied to each segment.

32. The drug injection method of Claim 22, further comprising:
resuming output of a control signal for the first speed-based injection sequence that was temporarily stopped when an operation based on the temporary basal injection mode is completed.

33. The drug injection method of Claim 22, further comprising:
resuming output of a control signal for the first speed-based injection sequence that was temporarily stopped when an operation based on the temporary basal injection mode is completed,
wherein an operation according to the first speed-based injection sequence which is scheduled to be performed during the pause period is omitted and an operation according to the first speed-based injection sequence which is scheduled to be performed immediately after the operation based on the temporary basal injection mode is completed is performed.

34. The drug injection method of Claim 22, further comprising:
receiving a user's activity level data or blood sugar level information from a program executed on the drug injection device or one or more wearable devices communicatively connected to the drug injection device when execution of the temporary basal injection mode is completed, and determining whether to continue the temporary basal injection mode based on the user's activity level data or blood sugar level information,
wherein the user's activity level data includes the user's heart rate or step count.

35. The drug injection method of Claim 24,
wherein the speed-based injection sequence is configured by arranging M number of pulse blocks (M is a natural number less than or equal to N) selected from among N number of pulse blocks (N is a natural number), which supply different amounts of drug, based on temporary basal injection conditions, and
the sum of durations of the M number of selected pulse blocks and a plurality of pause blocks following the pulse blocks is set to correspond to the drug injection period.

36. The drug injection method of Claim 35,
wherein the M number of pulse blocks of the speed-based injection sequence are set to satisfy the drug injection speed of basal injection conditions or the temporary basal injection conditions while ensuring that the total number of times the selected pulse blocks are used matches a predetermined number or a maximum number and a duration of the pause block following each pulse block is less than a recommended pause period.
